# FASCICULE DE BREVET EUROPEEN

(11) **EP 1 159 939 B1**
(45) Date de publication et mention de la délivrance du brevet: **20.07.2005**
(21) Numéro de dépôt: 01420111.5
(22) Date de dépôt: 17.05.2001
(51) Int. Cl.: A61F 2/42

(54) **Prothèse totale de poignet**
Handgelenktotalprothese
Total wrist prosthesis

(30) Priorité: 30.05.2000 FR 0006881
(43) Date de publication de la demande: 05.12.2001
(73) Titulaire: Biomet France, 26000 Valence (FR); Schernberg, François, 51100 Reims (FR)
(72) Inventeur: Schernberg, François, 51100 Reims (FR); de Witte, Gérard, 26000 Chateauneuf sur Isere (FR)
(74) Mandataire: Vuillermoz, Bruno

(56) Documents cités:
- EP-A- 0 338 715
- EP-A- 0 524 874
- EP-A- 0 532 440
- EP-A- 0 841 041
- WO-A-91/04718
- DE-A- 2 309 432
- DE-A- 4 433 483
- DE-B- 2 146 253
- FR-A- 2 314 702
- FR-A- 2 407 707
- FR-A- 2 450 600
- FR-A- 2 669 214
- FR-A- 2 712 180
- US-A- 4 063 314
- US-A- 4 307 473
- US-A- 5 776 203

## Description

L'invention concerne une prothèse totale de poignet.

Il est bien connu que les phénomènes d'arthrose des différents os constitutifs du poignet résultent soit du vieillissement naturel de ces os, soit d'une réaction post traumatique notamment en cas de fractures.

L'articulation du poignet peut être également le siège de manifestations articulaires inflammatoires caractéristiques chez les patients atteints de polyarthrite rhumatoïde. De façon connue, outre les phénomènes de douleur résultant de cette pathologie, on observe également d'importantes déformations pouvant conduire à une impotence fonctionnelle.

Le traitement des pathologies ci-avant décrites consiste avant tout, à administrer au sujet des anti-inflammatoires, puis bien souvent à procéder à la mise en place d'une prothèse totale de poignet.

A cet effet, le document EP-A-0 532 440 décrit une prothèse totale de poignet constituée de trois éléments articulés entre eux, l'élément intermédiaire étant libre en rotation dans deux plans perpendiculaires permettant le déplacement radio-cubital et dorso-palmaire du centre articulaire médiocarpien en cours de mouvement. Cependant, cette prothèse ne permet pas d'obtenir le mouvement de rotation dans un sens ou dans l'autre du poignet, par rapport au plan vertical, de sorte qu'elle ne reproduit pas la cinématique d'un poignet sain. Un autre inconvénient de cette prothèse est d'être sujette à la luxation de l'élément métacarpien par rapport à l'élément intermédiaire. On peut en outre observer un désancrage partiel du second élément par rapport au troisième métacarpien.

Le document FR-A-2 407 707 décrit une prothèse totale de poignet, constituée de trois éléments respectivement, un premier élément destiné à être ancré dans le radius, un second élément destiné à être ancré dans le troisième métacarpe, l'articulation du second élément sur le premier élément étant effectuée par le biais d'une rotule. Si cette prothèse permet de reproduire des mouvements biaxiaux ou triaxiaux, la rotule est en elle même dépourvue de tout degré de liberté augmentant ainsi le risque de douleur tout en diminuant la mobilité de l'ensemble.

En outre et de même que précédemment, on observe de possibles phénomènes de luxation sinon de désancrage de l'élément métacarpien.

Dans le document DE-A- 2 309 432, l'élément de liaison présente certes une tête sphérique, rotative et apte à coulisser sur un pivot, se logeant dans une calotte sphérique de l'élément carpien. Cependant, l'absence de moyen de maintien de la sphère dans la cavité est particulièrement propice à la luxation.

Dès lors, le problème que se propose de résoudre l'invention est de développer une prothèse totale de poignet ne présentant pas l'ensemble de ces inconvénients, mais au contraire, proposant les degrés de liberté sensiblement identiques à ceux de l'articulation, avec des débattements dans chacun de ces degrés de liberté proches de ceux de l'articulation.

L'invention a donc pour objet une prothèse totale de poignet comprenant un premier élément radial destiné à être ancré dans l'extrémité distale du radius, et un second élément carpien-métacarpien destiné à être ancré notamment au niveau de la deuxième rangée du carpe après ablation de la première rangée, lesdits premier et second éléments étant articulés par le biais d'une pièce deliaison. Ladite pièce de liaison présente des moyens aptes à coopérer avec des organes correspondant ménagés au niveau des éléments radial et carpien-métacarpien, conduisant à un mouvement :
- d'une part, de translation selon un plan sagittal, et de rotation par rapport à la direction de ladite translation de ladite pièce de liaison par rapport à l'élément radial ;
- et d'autre part, de flexion-extension de l'élément carpien-métacarpien par rapport à la pièce de liaison,
ledit mouvement permettant un effet de piston entre l'élément carpien-métacarpien et l'élément radial.

En outre, ladite pièce de liaison présente une tige prolongée à l'une de ses extrémités par une sphère ou rotule, destinée à coopérer avec une cavité hémisphérique de forme sensiblement correspondante ménagée au niveau de l'élément carpien-métacarpien.

Selon l'invention, ladite sphère est maintenue au sein de ladite cavité au moyen d'une bague munie d'un orifice traversant de diamètre inférieur à celui de la cavité hémisphérique, ladite bague étant solidaire de la face proximale de l'embase.

Cette bague permet d'éviter tout phénomène de luxation de la pièce de liaison par rapport au second élément, grâce au maintien de la sphère de ladite pièce de liaison dans la cavité hémisphérique de l'élément carpien-métacarpien.

Cette cavité hémisphérique, ainsi que la bague l'obturant partiellement présente avantageusement deux découples, ménagées à l'aplomb des zones de débattement en extension et en flexion, et ce, afin d'augmenter encore le débattement angulaire de la pièce de liaison, et par voie de conséquence de la prothèse selon ces deux degrés de liberté.

En d'autres termes, l'invention consiste à avoir modifié la structure de la pièce de liaison reliant l'élément radial à l'élément métacarpien d'une prothèse de poignet, modification structurale permettant de conduire à une mobilité supplémentaire non seulement selon le plan transversal mais également selon le plan sagittal, grâce à l'effet de piston provoqué par ladite pièce de liaison. Outre la mobilité supplémentaire, le double degré de liberté entre la pièce de liaison et le premier élément d'une part et le second élément d'autre part, permet d'augmenter le débattement limitant voire annulant ainsi tout désancrage du premier élément ou du second élément ainsi que tout risque de luxation. Elle permet en outre de disposer des degrés de liberté correspondant aux mouvements d'extension et de flexion de la main par rapport au bras, et ce, selon un débattement identique sinon proche des débattements naturels.

Selon une caractéristique avantageuse, l'ouverture de la bague présente un chanfrein permettant d'améliorer le débattement de l'élément carpien-métacarpien par rapport à la pièce de liaison.

Bien entendu, la mobilité supplémentaire obtenue dans le plan sagittal est limitée naturellement par les tendons et les muscles de l'articulation du poignet, étant rappelé que la pièce de liaison n'est reliée à aucune structure musculaire, ligamentaire ou osseuse.

Selon une autre caractéristique de l'invention, l'élément radial se présente sous forme d'un plateau solidaire d'une queue d'ancrage, ledit plateau venant en appui au niveau de l'extrémité distale du radius après résection.

En pratique, l'élément radial peut être réalisé en tout matériau connu tel que métal, alliage du type chrome ou cobalt, acier inox, céramique, etc...

Par ailleurs, la surface de la queue d'ancrage présente ou non des rainures permettant son positionnement avec ou sans ciment dans la diaphyse radiale.

Pour reproduire l'axe de déviation radio-cubital d'un poignet sain, lequel est en général décalé vers le cubitus, la queue d'ancrage est décalée par rapport au centre du plateau.

Pour coopérer avec les moyens correspondants de la pièce de liaison, la queue d'ancrage est munie d'un puits cylindrique et colinéaire avec la direction principale du radius.

Dans une forme de réalisation avantageuse, l'axe longitudinal du puits est décalé par rapport à l'axe longitudinal de la queue d'ancrage.

Selon une autre caractéristique de la prothèse de l'invention, l'élément carpien-métacarpien se présente sous la forme d'une embase comportant sur sa face distale des moyens d'ancrage aux os du carpe.

Dans une première forme de réalisation, les moyens d'ancrage se présentent sous forme d'au moins deux plots d'ancrage.

Avantageusement, la face distale est munie de trois plots d'ancrage respectivement deux plots latéraux destinés à être ancrés dans le trapézoïde et l'os crochu, un plot central de longueur supérieure destiné à être ancré dans le troisième métacarpien.

Dans une seconde forme de réalisation, les moyens d'ancrage se présentent sous forme d'au moins deux oeillets aptes à recevoir de vis de fixation, destinées à être vissées dans les os du métacarpien.

Dans une troisième forme de réalisation, les moyens d'ancrage se présentent sous forme d'au moins deux plots d'ancrage complétés par au moins deux vis de fixation.

De même que précédemment, le second élément est fabriqué dans tout matériau connu de l'homme du métier. Egalement, les pions d'ancrage présentent ou non des rainures améliorant l'efficacité de l'ancrage avec ou sans ciment.

Par ailleurs, pour augmenter l'extension de l'articulation des métacarpiens par rapport au radius, la face distale de l'embase du second élément supportant les moyens d'ancrage est inclinée d'un angle inférieur à 20° par rapport à sa face proximale.

S'agissant de la pièce de liaison, celle-ci est constituée d'une tige dont la longueur et la section correspondent sensiblement à celles du puit correspondant ménagé dans la queue du premier élément. Comme déjà dit, ladite tige est prolongée à l'une de ses extrémités par une sphère destinée à coopérer avec la cavité correspondante du second élément.

L'invention et les avantages qui en découlent ressortiront mieux des exemples de réalisation qui suivent à l'appui des figures annexées.
La figure 1 est une vue éclatée de l'ensemble des éléments constitutifs de la prothèse conforme à une première forme de réalisation de l'invention.
La figure 2 est une représentation schématique de la pièce de liaison.
Les figures 3a et 3b sont des représentations schématiques de l'élément radial, respectivement en section longitudinale et transversale.
Les figures 4a et 4b sont des représentations schématiques de l'élément métacarpien, respectivement en section longitudinale et en perspective.
Les figures 5a et 5b sont des représentations schématiques de la bague rendue solidaire de l'élément métacarpien, respectivement en vue du dessus et en section transversale.
La figure 6 est une représentation schématique de la prothèse conforme à la première forme de réalisation de l'invention montrée assemblée.
La figure 7 est une représentation schématique du positionnement anatomique de la prothèse.
Les figures 8a, 8b et 8c sont des représentations schématiques en perspective de la pièce de liaison et de l'élément métacarpien d'une seconde forme de réalisation de l'invention
Les figures 9a, 9b et 9c sont des représentations schématiques illustrant le débattement de la prothèse pour cette seconde forme de réalisation.

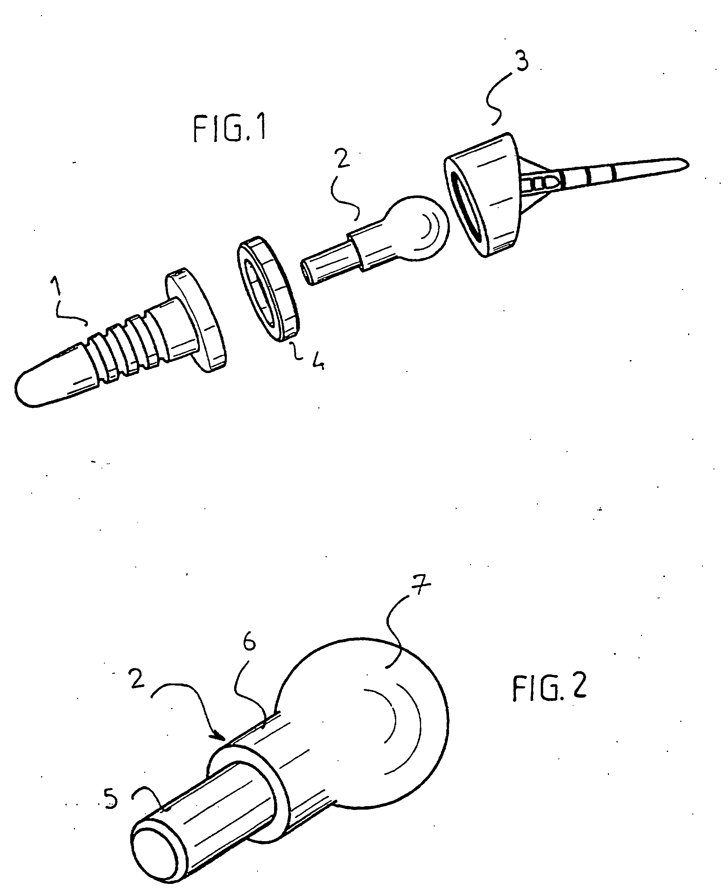

Comme le montre la figure 1, la prothèse totale de poignet de l'invention est constituée de quatre éléments, respectivement un premier élément radial (1), une pièce de liaison (2), un second élément carpien-métacarpien (3), le maintien de la pièce de liaison par rapport à l'élément carpien-métacarpien étant assuré par une bague (4).

La pièce de liaison (2) se présente sous forme d'une tige (5) munie sur partie de sa longueur d'une surépaisseur (6) se prolongeant par une tête sphérique (7), destinée à faire fonction de rotule.

La tige (5) est destinée à coopérer avec le puits (8) de la queue (9) constitutive de l'élément radial (1), tel que représenté en section sur la figure 3a, lequel est rendu solidaire du radius après résection partielle de celui-ci par le biais d'un plateau (10).

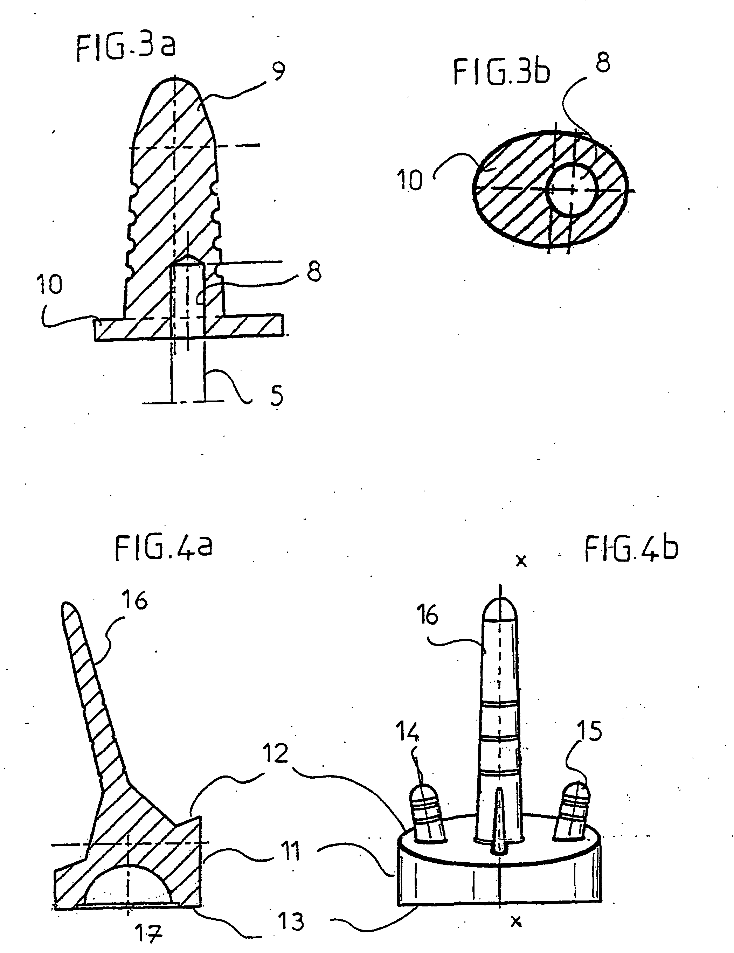

Comme le montrent cette figure ainsi que la figure 3b, la queue est décalée par rapport au centre du plateau, de même que l'axe longitudinal du puits (8) est décalé par rapport à celui de la queue (9) et ce, de sorte à reproduire l'axe de déviation radio-cubital d'un poignet sain.

Bien entendu, les caractéristiques dimensionnelles en terme de profondeur et de section du puits (8) de l'élément radial et de la tige (5) de l'élément de liaison sont identiques au jeu près.

Le plateau (10) est solidarisé notamment au moyen de ciment audit radius après résection partielle de celui-ci. De même, la queue d'ancrage est munie de rainures transversales destinées à renforcer sa fixation dans la diaphyse radiale.

Sur la figure 4, on a représenté le profil de l'élément métacarpien caractéristique de l'invention (figure 4b) ainsi qu'une section selon l'axe X-X de la figure 4b (figure 4a).

Comme le montre la figure 4b, l'élément carpien-métacarpien (3) est constitué d'une embase (11) de section arrondie, dont la face supérieure distale (12) supporte trois plots, respectivement deux plots latéraux (14, 15) orientés vers l'extérieur, destinés à être ancrés dans les os latéraux du carpe réséqués, à savoir le trapézoïde et l'os crochu, ainsi qu'un plot central (16) de longueur supérieure, destiné à être ancré dans le canal médullaire du troisième métacarpien, formant un angle compris entre 102 et 108° par rapport à la face inférieure proximale (13).

Selon une autre caractéristique et comme le montre la figure 4a, la face distale présente un angle d'environ 20° par rapport à la face inférieure proximale (13) et ce, de sorte à augmenter l'extension de l'articulation des métacarpes par rapport au radius.

Pour coopérer avec la tête sphérique (7) de la pièce de liaison (2), la face (13) proximale de l'embase présente en outre une cavité (17) hémisphérique dont le diamètre correspond sensiblement au diamètre de ladite tête sphérique de la pièce de liaison.

Enfin, pour permettre d'éviter toute luxation de la pièce de liaison par rapport à l'élément carpien-métacarpien (3), une bague (4) est solidarisée avec l'embase (11) de l'élément carpien-métacarpien (3).

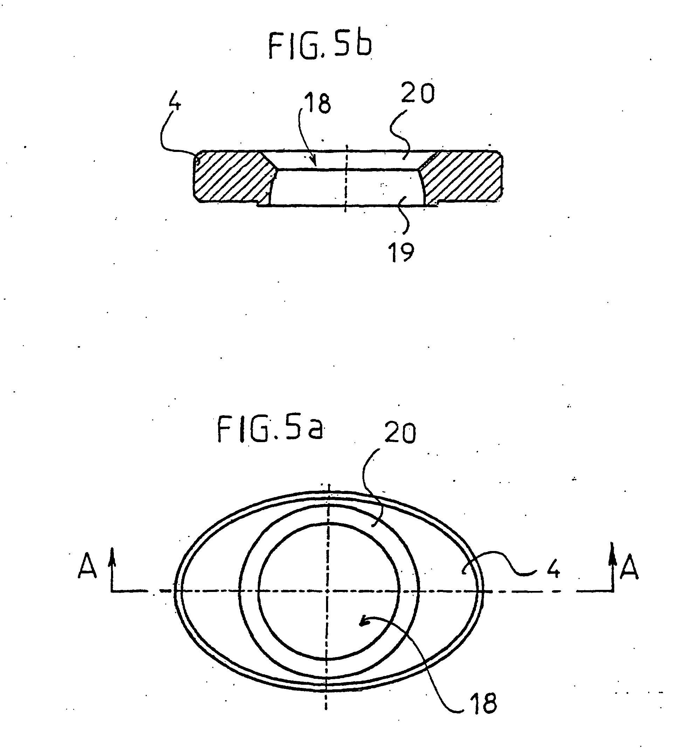

Cette bague est plus précisément représentée sur les figures 5a de profil, et 5b en section selon l'axe A-A de la figure 5a.

Cette bague est munie d'un orifice traversant (18), dont le diamètre est inférieur à celui de la sphère de la pièce de liaison (2). Ainsi, l'orifice de la bague (4) présente une première portion décroissante (19) destiné à coopérer avec la partie de la sphère (7) de diamètre correspondant et une seconde portion croissante (20) constituant un chanfrein permettant d'augmenter le degré de débattement de l'élément carpien-métacarpien par rapport à l'élément de liaison.

Bien entendu, la forme générale de la bague correspond à celle de l'embase de manière à être ajustée précisément.

Pour mettre en place la prothèse, on procède tout d'abord à l'ablation de la première rangée de carpe, puis on résèque à la fois le grand os, l'os crochu et le trapézoïde afin d'ancrer l'embase (13), puis les plots (14, 15). On creuse également le canal médullaire du troisième métacarpien de sorte à venir y ancrer le troisième plot (16).

On résèque ensuite le radius afin de positionner l'élément radial, dont la queue d'ancrage est fixée dans la diaphyse radiale, le plateau venant en appui contre l'extrémité distale du radius.

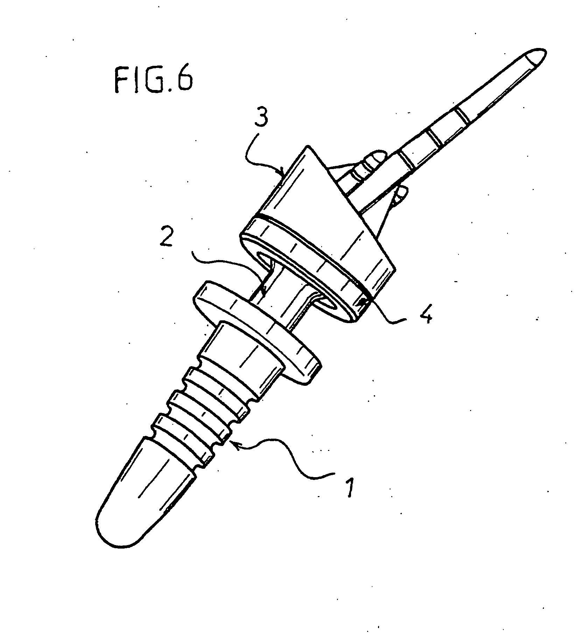

La figure 6 montre la coopération des différents éléments de la prothèse entre eux.

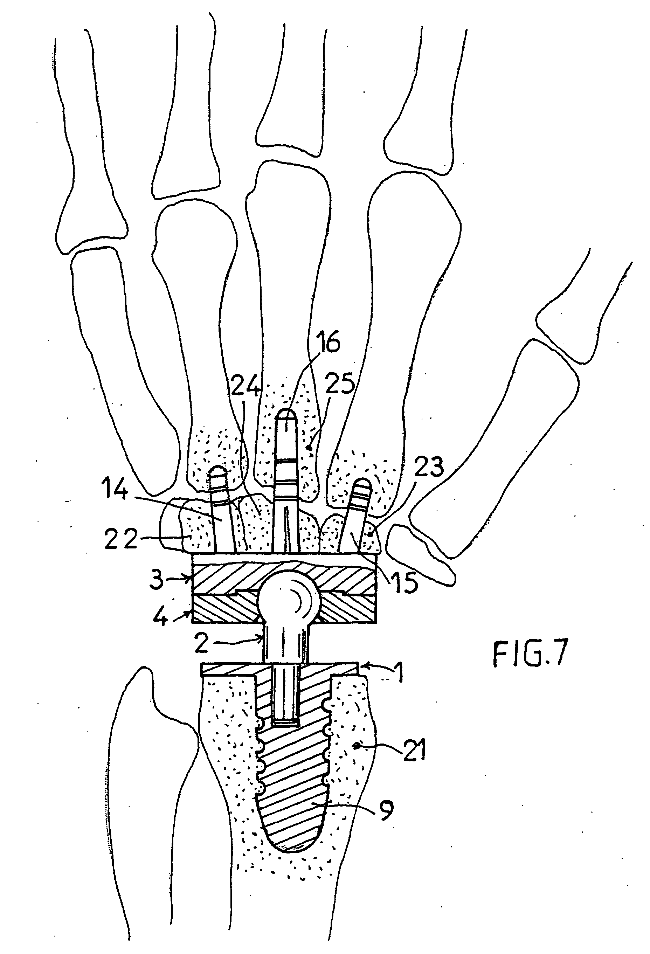

Sur la figure 7, on a représenté schématiquement le positionnement anatomique de la prothèse de l'invention.

Comme le montre cette figure, la queue (9) de l'élément radial (1) est ancrée dans le radius (21). S'agissant de l'élément carpien-métacarpien (3), celui-ci est ancré dans l'os crochu (22), le trapézoïde (23), le grand os (24) et le canal médullaire du troisième métacarpien (25), par le biais des plots latéraux (14, 15) et du plot central (16). L'élément carpien-métacarpien, solidaire de la pièce de liaison, effectue un mouvement de piston par rapport à l'élément radial permettant ainsi d'optimiser l'articulation du poignet.

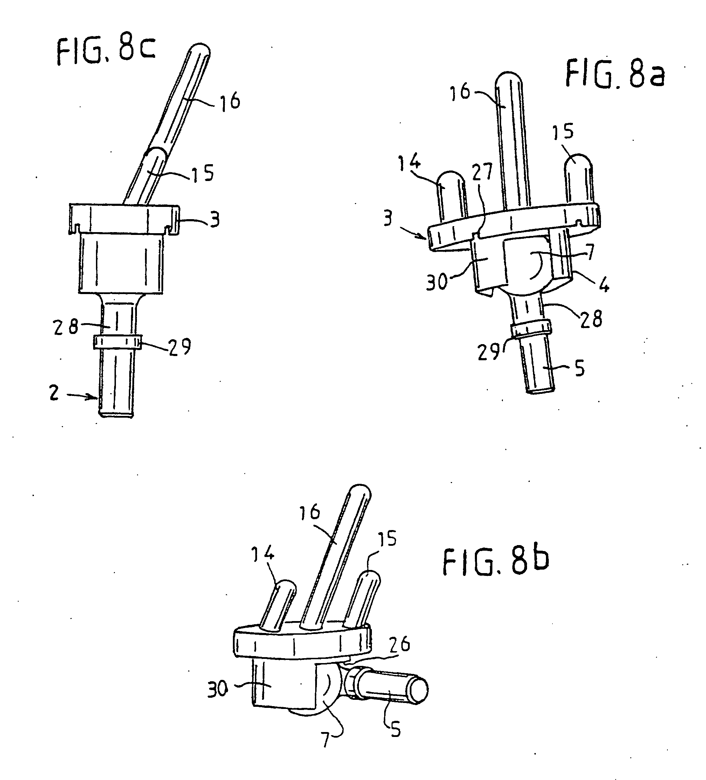

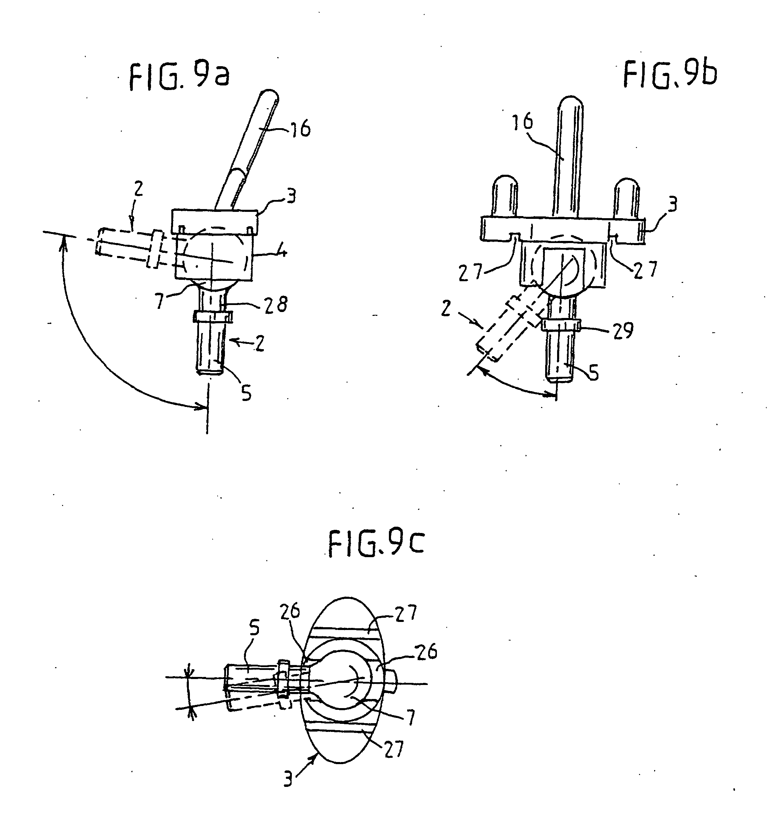

On a représenté en relation avec les figures 8 et 9 une seconde forme de réalisation de l'invention. Dans celle-ci, la zone de réception de la tête (7) de la pièce de liaison (2) au sein de l'élément métacarpien (3) comporte une base (30), s'étendant depuis la face proximale (13) dudit élément, et définit avec la bague (4), comme dans l'exemple de réalisation précédent, une cavité hémisphérique. Mais cette base (30), ainsi que la bague (4) présentent deux découpes ou lumières (26), sensiblement diamétralement opposées, et s'étendant sensiblement sur toute la hauteur de ladite cavité hémisphérique. Ce faisant, ces lumières dégagent une zone de débattement supplémentaire pour la pièce de liaison. Celle-ci présente d'ailleurs à cet effet, une zone plus fine (28), destinée à coopérer avec lesdites lumières (30), sensiblement de même diamètre que la tige (5), et séparée de celle-ci par un anneau (29), destiné à limiter la course de la tige (5) au sein du puits (8) de l'élément radial (1).

On a ainsi représenté au sein des figures 8b et 9a - 9c les différentes possibilités de débattement de la pièce de liaison (2) par rapport à l'élément métacarpien. Lorsque la tige de la pièce de liaison se trouve à l'aplomb des lumières (26), un débattement de l'ordre de 98° peut être obtenu, alors que dans les autres situations, seul un débattement de l'ordre de 38° est disponible, dans le plan perpendiculaire à l'embase de l'élément métacarpien.

Avantageusement, la face proximale (13) de l'élément métacarpien présente deux rainures (27), destinées à recevoir un câble ou moyen équivalent. Ce ou ces câbles sont en outre passés ensuite entre les os des phalanges ou du métacarpe situés à l'aplomb, dans l'objectif de sécuriser l'ancrage de l'élément métacarpien, et par voie de conséquence de la prothèse au sein de l'articulation.

L'invention et les avantages qui en découlent ressortirent bien de la description.

On notera en particulier la mobilité importante obtenue par la prothèse de l'invention, grâce à l'effet piston obtenu entre les éléments carpien-métacarpien et radial, tout en évitant tout risque de luxation et de désancrage.

On notera également l'optimisation des débattements possibles selon chacun des degrés de liberté offerts.

## Revendications

1. Prothèse totale de poignet comprenant un premier élément radial (1) destiné à être ancré dans l'extrémité distale du radius, et un second élément carpien-métacarpien (3), destiné à être ancré notamment au niveau de la deuxième rangée du carpe après ablation de la première rangée, lesdits premier et second éléments étant articulés par le biais d'une pièce de liaison (2), ladite pièce de liaison (2) présentant des moyens aptes à coopérer avec des organes correspondants ménagés au niveau des éléments radial (1) et carpien-métacarpien (3), conduisant à un mouvement :
• d'une part, de translation selon un plan sagittal, et de rotation par rapport à la direction de ladite translation de la pièce de liaison par rapport à l'élément radial,
• et d'autre part, de flexion-extension de l'élément carpien-métacarpien par rapport à la pièce de liaison,
ledit mouvement permettant un effet de piston de l'élément carpien-métacarpien par rapport à l'élément radial,
ladite pièce de liaison (2) présentant une tige (5) prolongée (6) à l'une de ses extrémités par une sphère (7) destinée à coopérer avec une cavité hémisphérique (17) de forme sensiblement correspondante, ménagée au niveau de l'élément carpien-métacarpien (3),
***caractérisée* en ce que** ladite sphère (7) est maintenue au sein de ladite cavité (17) au moyen d'une bague (4) solidaire de la face proximale (13) de l'embase (11) de l'élément carpien-métacarpien (3), ladite bague étant munie d'un orifice traversant (18) de diamètre inférieur à celui de la cavité hémisphérique (17).

2. Prothèse de poignet selon la revendication 1, ***caractérisée* en ce que** l'ouverture de la bague (4) présente en outre un chanfrein (20) permettant d'améliorer le débattement de l'élément carpien-métacarpien (3) par rapport à la pièce de liaison (2).

3. Prothèse de poignet selon l'une des revendications 1 ou 2, ***caractérisée* en ce que** la zone de réception de la tête (7) de la pièce de liaison (2) au sein de l'élément métacarpien (3) comporte une base (30), s'étendant depuis la face proximale (13) dudit élément, et définit avec la bague (4), une cavité hémisphérique destinée à recevoir ladite tête(7), et **en ce que** la base (30) ainsi que la bague (4) présentent deux découpes ou lumières (26), sensiblement diamétralement opposées, et s'étendant sensiblement sur toute la hauteur de ladite cavité hémisphérique, de telle sorte à définir une zone de débattement supplémentaire pour la pièce de liaison (2).

4. Prothèse de poignet selon la revendication 3, ***caractérisée* en ce que** la pièce de liaison présente une zone plus fine (28), destinée à coopérer avec lesdites lumières (26), sensiblement de même diamètre que la tige (5), et séparée de celle-ci par un anneau (29), destiné à limiter la course de la tige (5) au sein du puits (8) de l'élément radial (1).

5. Prothèse de poignet selon l'une des revendications 1 à 4, ***caractérisée* en ce que** l'élément radial (1) se présente sous la forme d'un plateau solidaire d'une queue d'ancrage, ledit plateau (10) venant en appui au niveau de l'extrémité distale du radius après résection.

6. Prothèse de poignet selon la revendication 5, ***caractérisée* en ce que** la queue d'ancrage (9) est décalée par rapport au centre du plateau (10).

7. Prothèse de poignet selon la revendication 6, ***caractérisée* en ce que** l'élément radial (1) présente des moyens de coopération avec la pièce de liaison (2), constitués d'un puits (8) cylindrique et perpendiculaire au plateau (10).

8. Prothèse de poignet selon la revendication 7, ***caractérisée* en ce que** l'axe longitudinal du puits (8) est décalé par rapport à l'axe longitudinal de la queue d'encrage (9).

9. Prothèse de poignet selon l'une des revendications 1 à 8, ***caractérisée* en ce que** l'élément carpien-métacarpien (3) comporte sur la face distale (12) de son embase (11) des moyens d'ancrage aux os de carpe.

10. Prothèse de poignet selon la revendication 9, ***caractérisée* en ce que** les moyens d'ancrage se présentent sous forme de trois plots d'ancrage, respectivement deux plots latéraux (14, 15) destinés à être ancrés dans le trapézoïde et l'os crochu, et un plot central (16), de longueur supérieure, destiné à être ancré dans le troisième métacarpien.

11. Prothèse de poignet selon la revendication 9, ***caractérisée* en ce que** les moyens d'ancrage se présentent sous forme d'au moins deux oeillets aptes à recevoir des vis de fixation, destinées à être vissées dans les métacarpiens.

12. Prothèse de poignet selon la revendication 9, ***caractérisée* en ce que** la face distale (12) de l'embase (11) supportant les moyens d'ancrage est inclinée d'un angle inférieur à 20° par rapport à sa face proximale (13).

13. Prothèse de poignet selon l'une des revendications 7 à 12, ***caractérisée* en ce que** la tige (5) de la pièce de liaison (2) présente une longueur et une section correspondant sensiblement à celles du puits (8) correspondant ménagé dans la queue (9) de l'élément radial (1), au sein duquel ladite tige est destinée à venir s'insérer.

14. Prothèse de poignet selon l'une des revendications 3 à 13, ***caractérisée* en ce que** la face proximale (13) de l'élément métacarpien (3) présente deux rainures (27), destinées à recevoir un câble ou moyen équivalent, destiné à être passés entre les os des phalanges ou du métacarpe situés à l'aplomb, dans l'objectif de sécuriser l'ancrage de l'élément métacarpien, et par voie de conséquence de la prothèse au sein de l'articulation.

## Patentansprüche

1. Handgelenk-Totalendoprothese mit einem ersten radialen Element (1), welches zur Verankerung in dem distalen Ende des Radius bestimmt ist, und einem zweiten karpo-metakarpalen Element (3), welches zur Verankerung insbesondere im Bereich der zweiten Knochenreihe der Handwurzel nach Entfernung der ersten Reihe bestimmt ist, wobei das genannte erste und zweite Element über ein Verbindungsstück (2) aneinander angelenkt sind, wobei das genannte Verbindungsstück (2) Mittel aufweist, welche zum Zusammenwirken mit den entsprechenden im Bereich des radialen (1) und des karpo-metakarpalen Elements (3) angebrachten Organen geeignet sind, was zu folgenden Bewegungen führt:
◆ einerseits einer Translation entlang einer Sagittalebene und einer Rotation in bezug auf die Richtung der genannten Translation des Verbindungsstücks in bezug auf das radiale Element,
◆ und andererseits einer Beuge-Streck-Bewegung des karpo-metakarpalen Elements in bezug auf das Verbindungsstück,
wobei die genannte Bewegung eine Kolbenwirkung des karpo-metakarpalen Elements in bezug auf das radiale Element ermöglicht,
wobei das genannte Verbindungsstück (2) einen Schaft (5) aufweist, welcher an einem seiner Enden durch eine Kugel (7) verlängert (6) ist, welche zum Zusammenwirken mit einer halbkugelförmigen Vertiefung (17) von im wesentlichen entsprechender Form bestimmt ist, die im Bereich des karpo-metakarpalen Elements (3) angebracht ist,
**dadurch gekennzeichnet, daß** die genannte Kugel (7) mittels eines mit der proximalen Fläche (13) des Sockels (11) des karpo-metakarpalen Elements (3) fest verbundenen Rings innerhalb der genannten Vertiefung (17) gehalten ist,
wobei der genannte Ring mit einer durchgehenden Öffnung (18) versehen ist, deren Durchmesser kleiner ist als der der halbkugelförmigen Vertiefung (17).

2. Handgelenk-Prothese nach Anspruch 1, **dadurch gekennzeichnet, daß** die Öffnung des Rings (4) zudem eine Fase (20) aufweist, welche eine Verbesserung der Auslenkung des karpo-metakarpalen Elements (3) in bezug auf das Verbindungsstück (2) ermöglicht.

3. Handgelenk-Prothese nach einem der Ansprüche 1 oder 2, **dadurch gekennzeichnet, daß** die Zone zur Aufnahme des Kopfes (7) des Verbindungsstücks (2) innerhalb des metakarpalen Elements (3) eine Basis (30) umfaßt, die sich ausgehend von der proximalen Fläche (13) des genannten Elements erstreckt und mit dem Ring (4) eine halbkugelförmige Vertiefung bildet, welche zur Aufnahme des genannten Kopfes (7) bestimmt ist, und daß die Basis (30) sowie der Ring (4) zwei Einschnitte oder Schlitzlöcher (26) aufweisen, welche im wesentlichen diametral gegenüberliegend sind und sich im wesentlichen über die gesamte Höhe der genannten halbkugelförmigen Vertiefung erstrecken, so daß sie eine zusätzliche Auslenkungszone für das Verbindungsstück (2) bilden.

4. Handgelenk-Prothese nach Anspruch 3, **dadurch gekennzeichnet, daß** das Verbindungsstück eine dünnere Zone (28) aufweist, welche zum Zusammenwirken mit den genannten Schlitzlöchern (26) bestimmt ist, im wesentlichen den gleichen Durchmesser wie der Schaft (5) aufweist und von diesem durch einen Ring (29) getrennt ist, welcher dazu bestimmt ist, die Bewegung des Schaftes (5) innerhalb der Bohrung (8) des radialen Elements (1) zu begrenzen.

5. Handgelenk-Prothese nach einem der Ansprüche 1 bis 4, **dadurch gekennzeichnet, daß** das radiale Element (1) die Form einer mit einem Verankerungsschaft fest verbundenen Platte aufweist, wobei die genannte Platte (10) im Bereich des distalen Endes des Radius nach der Resektion aufliegt.

6. Handgelenk-Prothese nach Anspruch 5, **dadurch gekennzeichnet, daß** der Verankerungsschaft (9) in bezug auf die Mitte der Platte (10) versetzt ist.

7. Handgelenk-Prothese nach Anspruch 6, **dadurch gekennzeichnet, daß** das radiale Element (1) Mittel zum Zusammenwirken mit dem Verbindungsstück (2) aufweist, welche aus einer zylindrischen und zur Platte (10) rechtwinkligen Bohrung (8) bestehen.

8. Handgelenk-Prothese nach Anspruch 7, **dadurch gekennzeichnet, daß** die Längsachse der Bohrung (8) in bezug auf die Längsachse des Verankerungsschafts (9) versetzt ist.

9. Handgelenk-Prothese nach einem der Ansprüche 1 bis 8, **dadurch gekennzeichnet, daß** das karpo-metakarpale Element (3) auf der distalen Fläche (12) seines Sockels (11) Mittel zur Verankerung in den Handwurzelknochen umfaßt.

10. Handgelenk-Prothese nach Anspruch 9, **dadurch gekennzeichnet, daß** die Verankerungsmittel die Form dreier Verankerungsstifte aufweisen, nämlich zweier seitlicher Stifte (14, 15), bestimmt zur Verankerung im Trapezbein bzw. dem Hakenbein, und eines mittlerem Stiftes (16) von größerer Länge, bestimmt zur Verankerung in dem dritten Mittelhandknochen.

11. Handgelenk-Prothese nach Anspruch 9, **dadurch gekennzeichnet, daß** die Verankerungsmittel die Form mindestens zweier Ösen aufweisen, welche zur Aufnahme von Befestigungsschrauben geeignet sind, welche zum Einschrauben in die Mittelhandknochen bestimmt sind.

12. Handgelenk-Prothese nach Anspruch 9, **dadurch gekennzeichnet, daß** die distale Seite (12) des Sockels (11), welche die Verankerungsmittel trägt, um einen Winkel von weniger als 20° in bezug auf seine proximale Fläche (13) geneigt ist.

13. Handgelenk-Prothese nach einem der Ansprüche 7 bis 12, **dadurch gekennzeichnet, daß** der Schaft (5) des Verbindungsstücks (2) eine Länge und einen Querschnitt aufweist, welche im wesentlichen denen der entsprechenden Bohrung (8) entsprechen, die im Verankerungsschaft (9) des radialen Elements (1) angebracht ist und in der der genannte Schaft eingesetzt werden soll.

14. Handgelenk-Prothese nach einem der Ansprüche 3 bis 13, **dadurch gekennzeichnet, daß** die proximale Seite (13) des metakarpalen Elements (3) zwei Nuten (27) aufweist, welche zur Aufnahme eines Drahts oder gleichwertigen Mittels bestimmt sind, der bzw. das dazu bestimmt ist, zwischen den fluchtenden Knochen der Fingerglieder oder der Mittelhand geführt zu werden mit dem Ziel, die Verankerung des metakarpalen Elements und folglich der Prothese innerhalb des Gelenks zu sichern.

## Claims

1. Total wrist prosthesis, comprising a first radial element (1) intended to be anchored in the distal end of the radius, and a second carpal-metacarpal element (3) intended to be anchored in particular in the second carpal row after ablation of the first row, said first and second elements being articulated by way of a connection piece (2), said connection piece (2) having means which are able to cooperate with corresponding members formed on the radial (1) and carpal-metacarpal (3) elements, leading to a movement:
• firstly, of translation in a sagittal plane, and of rotation relative to the direction of said translation, of the connection piece with respect to the radial element,
• and, secondly, of flexion/extension of the carpal-metacarpal element with respect to the connection piece,
said movement permitting a piston effect of the carpal-metacarpal element with respect to the radial element,
said connection piece (2) having a rod (5) continued (6) at one of its ends by a ball (7) intended to cooperate with a hemispherical cavity (17) of substantially corresponding shape formed in the carpal-metacarpal element (3),
**characterized in that** said ball (7) is held within said cavity (17) by a washer (4) integral with the proximal face (13) of the seat (11) of the carpal-metacarpal element (3), said washer being provided with a through-opening (18) whose diameter is smaller than that of the hemispherical cavity (17).

2. Wrist prosthesis according to Claim 1,
**characterized in that** the opening of the washer (4) additionally has a bevel (20) making it possible to improve the clearance of the carpal-metacarpal element (3) with respect to the connection piece (2).

3. Wrist prosthesis according to either of Claims 1 and 2, **characterized in that** the zone in which the head (7) of the connection piece (2) is received within the metacarpal element (3) comprises a base (30), extending from the proximal face (13) of said element, and defines with the washer (4) a hemispherical cavity intended to receive said head (7), and **in that** the base (30) and the washer (4) have two cutouts or slots (26) which are arranged substantially diametrically opposite one another and extend substantially along the entire height of said hemispherical cavity, in such a way as to define a supplementary clearance zone for the connection piece (2).

4. Wrist prosthesis according to Claim 3, **characterized in that** the connection piece has a thinner zone (28) which is intended to cooperate with said slots (26) and is substantially of the same diameter as the rod (5), and separated from the latter by a ring (29) intended to limit the travel of the rod (5) inside the well (8) of the radial element (1).

5. Wrist prosthesis according to one of Claims 1 to 4, **characterized in that** the radial element (1) is in the form of a plate integral with an anchoring stem, said plate (10) bearing on the distal end of the radius after resection.

6. Wrist prosthesis according to Claim 5, **characterized in that** the anchoring stem (9) is offset with respect to the centre of the plate (10).

7. Wrist prosthesis according to Claim 6, **characterized in that** the radial element (1) has means which cooperate with the connection piece (2) and are composed of a cylindrical well (8) perpendicular to the plate (10).

8. Wrist prosthesis according to Claim 7, **characterized in that** the longitudinal axis of the well (8) is offset with respect to the longitudinal axis of the anchoring stem (9).

9. Wrist prosthesis according to one of Claims 1 to 8, **characterized in that** the carpal-metacarpal element (3) has, on the distal face (12) of its seat (11), means for anchoring in the carpal bones.

10. Wrist prosthesis according to Claim 9, **characterized in that** the anchoring means are in the form of three anchoring pins, namely two lateral pins (14, 15) intended to be anchored in the trapezoid and the hamate bone, and a central pin (16), of greater length, intended to be anchored in the third metacarpal.

11. Wrist prosthesis according to Claim 9, **characterized in that** the anchoring means are in the form of at least two eyelets which are able to receive fixation screws intended to be screwed into the metacarpals.

12. Wrist prosthesis according to Claim 9, **characterized in that** the distal face (12) of the seat (11) supporting the anchoring means is inclined by an angle of less than 20° with respect to its proximal face (13).

13. Wrist prosthesis according to one of Claims 7 to 12, **characterized in that** the rod (5) of the connection piece (2) has a length and a cross section corresponding substantially to those of the corresponding well (8) formed in the stem (9) of the radial element (1), within which well (8) said rod is intended to be inserted.

14. Wrist prosthesis according to one of Claims 3 to 13, **characterized in that** the proximal face (13) of the metacarpal element (3) has two grooves (27) intended to receive a cable or equivalent means intended to be passed between the bones of the phalanges or of the metacarpal situated in line therewith, the aim being to secure the anchoring of the metacarpal element and, consequently, of the prosthesis in the joint.
